# EUROPEAN PATENT APPLICATION

(11) **EP 4 679 091 A1**
(43) Date of publication of application: **14.01.2026**
(21) Application number: 24766809.8
(22) Date of filing: 15.02.2024
(51) Int. Cl.: G01N 33/543

(54) **ANALYSIS CHIP, ANALYSIS METHOD FOR SPECIMEN, AND ANALYSIS SYSTEM FOR SPECIMEN**

(30) Priority: 09.03.2023 JP 2023036622
(71) Applicant: WASEDA UNIVERSITY, Shinjuku-ku Tokyo 169-8050 (JP)
(72) Inventor: ITO, Etsuro, Tokyo 169-8050 (JP)
(74) Representative: Schmidt, Christian
(86) International application number: PCT/JP2024/005200
(87) International publication number: WO 2024/185437

(57) **Abstract**

An analysis chip includes: a specimen introduction portion into which a liquid specimen containing a protein or nucleic acid is introduced; a washing solution storage portion; a dissolving solution storage portion; a reaction portion provided with an immobilized antibody or nucleic acid; a first flow path that connects the specimen introduction portion and the reaction portion; a second flow path that connects the washing solution storage portion and the reaction portion; and a third flow path that connects the dissolving solution storage portion and the reaction portion and that includes a dry reagent enclosing portion provided midway in the flow path, the dry reagent enclosing portion enclosing a dry reagent containing NADH, a substrate for the enzyme in the enzyme-labeled antibody, thio-NAD, and dehydrogenase. By applying a predetermined centrifugal force to the analysis chip, analysis of a specimen by an ELISA method is performed.

## Description

### TECHNICAL FIELD

The present invention relates to an analysis chip, an analysis method for a specimen, and an analysis system for a specimen. More specifically, the present invention relates to an analysis chip capable of analyzing a protein or nucleic acid as a specimen, an analysis method for a specimen using the analysis chip, and an analysis system for a specimen including the analysis chip as a part of a configuration.

### BACKGROUND ART

For diagnosis of cancer, infectious diseases, or the like, it is sometimes required to detect a specific protein or nucleic acid in a specimen.

One method for detecting or quantifying proteins or nucleic acids is an enzyme-linked immunosorbent assay method (ELISA method). The ELISA method is a method of using highly specific antigen-antibody reactions to detect a color development (or luminescence) based on an enzyme reaction as a signal.

In the conventional ELISA method, attempting to perform highly sensitive measurement of trace amounts of proteins or nucleic acids requires efforts to detect weak signals. However, detecting such weak signals requires, for example, tasks such as using a high-sensitivity camera or accumulating signals over a long period of time, which imposes limitations on achieving higher sensitivity.

In order to overcome the above-described limitations, Etsuro Ito and others have developed an ultra-high sensitivity ELISA method, which combines the ELISA method with an enzyme cycling method and enables the highly sensitive measurement of trace amounts of proteins or nucleic acids, and have acquired patents (Patent Documents 1, 2, and others).

### RELATED DOCUMENT

### PATENT DOCUMENT

Patent Document 1: Japanese Patent No. 5265816
Patent Document 2: Japanese Patent No. 5500985

### SUMMARY OF THE INVENTION

### TECHNICAL PROBLEM

In Examples of Patent Documents 1 and 2, the specimen is analyzed through operations such as dispensing a reaction test solution into a microplate.

However, such analysis of the specimen described above typically needs to be performed in a laboratory or a hospital equipped with necessary facilities. That is, the analysis methods specifically described in Patent Documents 1 and 2 are ultra-high sensitive, but there is room for improvement in terms of simplicity and rapidity (as analysis cannot be performed immediately after specimen collection), and the analysis methods are not easily applicable in small clinics or mass screening settings in the current form.

The present invention has been made in view of such circumstances. One object of the present invention is to provide a unit capable of easily and rapidly analyzing a protein or nucleic acid.

### SOLUTION TO PROBLEM

The present inventors have completed the invention provided below and solved the above-described problems.

1. An analysis chip including:
   a specimen introduction portion into which a liquid specimen containing a protein or nucleic acid labeled with an enzyme-labeled antibody, or a liquid specimen containing an enzyme-labeled antibody and a protein or nucleic acid is introduced;
   a washing solution storage portion;
   a dissolving solution storage portion;
   a reaction portion provided with an immobilized antibody or nucleic acid;
   a first flow path that connects the specimen introduction portion and the reaction portion;
   a second flow path that connects the washing solution storage portion and the reaction portion; and
   a third flow path that connects the dissolving solution storage portion and the reaction portion and that includes a dry reagent enclosing portion provided midway in the flow path, the dry reagent enclosing portion enclosing a dry reagent containing NADH, a substrate for an enzyme in the enzyme-labeled antibody, thio-NAD, and dehydrogenase,
   in which, by making the first flow path, the second flow path, and the third flow path different from each other in at least one of a cross-sectional area, a length, and hydrophilicity of a flow path inner wall,
   when a predetermined centrifugal force is applied to the analysis chip: (1) the liquid specimen introduced into the specimen introduction portion is first caused to reach the reaction portion; after the specimen reaches the reaction portion, (2) a washing solution introduced into the washing solution storage portion is caused to reach the reaction portion; and after the washing solution reaches the reaction portion, (3) a solution, in which the dry reagent has been dissolved in a dissolving solution introduced into the dissolving solution storage portion, is caused to reach the reaction portion.
2. The analysis chip according to 1.,
   in which the first flow path, the second flow path, and the third flow path are different from each other in at least one of the cross-sectional area and the hydrophilicity of the flow path inner wall, and
   a magnitude of the predetermined centrifugal force increases at least once as time elapses.
3. The analysis chip according to 1.,
   in which the first flow path, the second flow path, and the third flow path are different from each other in at least the length, and
   a magnitude of the predetermined centrifugal force is substantially constant over time.
4. An analysis chip including:
   a specimen introduction portion into which a liquid specimen containing a protein or nucleic acid labeled with an enzyme-labeled antibody, or a liquid specimen containing an enzyme-labeled antibody and a protein or nucleic acid is introduced;
   a washing solution storage portion;
   a dissolving solution storage portion;
   a reaction portion provided with an immobilized antibody or nucleic acid;
   a first flow path that connects the specimen introduction portion and the reaction portion;
   a second flow path that connects the washing solution storage portion and the reaction portion; and
   a third flow path that connects the dissolving solution storage portion and the reaction portion and that includes a dry reagent enclosing portion provided midway in the flow path, the dry reagent enclosing portion enclosing a dry reagent containing NADH, a substrate for an enzyme in the enzyme-labeled antibody, thio-NAD, and dehydrogenase,
   in which at least one of the second flow path and the third flow path includes a blocking portion provided midway, and the blocking portion is configured to prevent a liquid from passing through, and
   the blocking portion is destroyable by light or heat, and the liquid is allowed to pass through the flow path when the blocking portion is destroyed.
5. The analysis chip according to any one of 1. to 4.,
   in which the second flow path includes three to five sub flow paths a that connect the washing solution storage portion and the reaction portion, and
   the sub flow paths are different from each other in at least one of a cross-sectional area, a length, and hydrophilicity of a flow path inner wall.
6. The analysis chip according to any one of 1. to 5.,
   in which the third flow path includes two sub flow paths, a sub flow path b1 and a sub flow path b2, extending from the dissolving solution storage portion, a mixing portion to which the sub flow path b1 and the sub flow path b2 are connected, a sub flow path b3 that connects the mixing portion to the reaction portion, a first dry reagent enclosing portion c1 provided midway in the sub flow path b1, and a second dry reagent enclosing portion c2 provided midway in the sub flow path b2, and
   one of the dry reagent enclosing portion c1 and the dry reagent enclosing portion c2 encloses NADH and a substrate for the enzyme in the enzyme-labeled antibody, and the other of the dry reagent enclosing portion c1 and the dry reagent enclosing portion c2 encloses thio-NAD and dehydrogenase.
7. The analysis chip according to any one of 1. to 6., further including:
   a waste liquid storage portion; and
   a fourth flow path that connects the reaction portion and the waste liquid storage portion.
8. The analysis chip according to any one of 1. to 7., further including:
   a washing solution package in which the washing solution is enclosed with a first packaging material,
   in which the washing solution package is configured such that the washing solution is supplied to the washing solution storage portion when the first packaging material is destroyed.
9. The analysis chip according to 8.,
   in which the washing solution is a buffer solution containing a surfactant.
10. The analysis chip according to any one of 1. to 9., further including:
   a dissolving solution package in which the dissolving solution is enclosed with a second packaging material,
   in which the dissolving solution package is configured such that the dissolving solution is supplied to the dissolving solution storage portion when the second packaging material is destroyed.
11. The analysis chip according to 10.,
   in which the dissolving solution is a buffer solution.
12. An analysis method for a specimen, including:
   a step of introducing a liquid specimen containing a protein or nucleic acid labeled with an enzyme-labeled antibody into the specimen introduction portion in the analysis chip according to any one of 1 to 3;
   a step of applying a centrifugal force to the analysis chip to: (1) first cause the specimen to reach the reaction portion; after the specimen reaches the reaction portion, (2) cause a washing solution introduced into the washing solution storage portion to reach the reaction portion and discharge the washing solution from the reaction portion; and then (3) cause a solution, in which the dry reagent has been dissolved in a dissolving solution introduced into the dissolving solution storage portion, to reach the reaction portion; and
   a step of measuring an absorbance of a liquid stored in the reaction portion.
13. An analysis method for a specimen, including:
   a step of introducing a liquid specimen containing a protein or nucleic acid labeled with an enzyme-labeled antibody into the specimen introduction portion in the analysis chip according to 4.;
   a step of applying a centrifugal force to the analysis chip and destroying the blocking portion using light or heat to: (1) first cause the specimen to reach the reaction portion; after the specimen reaches the reaction portion, (2) cause a washing solution introduced into the washing solution storage portion to reach the reaction portion and discharge the washing solution from the reaction portion; and then (3) cause a solution, in which the dry reagent has been dissolved in a dissolving solution introduced into the dissolving solution storage portion, to reach the reaction portion; and
   a step of measuring an absorbance of a liquid stored in the reaction portion.
14. An analysis system for a specimen, including:
   the analysis chip according to any one of 1. to 3.;
   a centrifugal force applying unit configured to apply a centrifugal force to the analysis chip; and
   an absorbance measuring unit configured to measure an absorbance of a liquid stored in the reaction portion in the analysis chip.
15. An analysis system for a specimen, including:
   the analysis chip according to 4.;
   a centrifugal force applying unit configured to apply a centrifugal force to the analysis chip;
   a light source or a heat source configured to destroy the blocking portion in the analysis chip; and
   an absorbance measuring unit configured to measure an absorbance of a liquid stored in the reaction portion in the analysis chip.

### ADVANTAGEOUS EFFECTS OF INVENTION

According to the present invention, it is possible to easily and rapidly analyze a protein or nucleic acid.

### BRIEF DESCRIPTION OF THE DRAWINGS

[FIGS. 1A and 1B] Diagrams illustrating an analysis chip of a first embodiment.
[FIG. 2] A diagram illustrating the analysis chip of the first embodiment.
[FIG. 3] A diagram illustrating movement of various liquids in the analysis chip of the first embodiment.
[FIG. 4] A diagram illustrating an aspect of a mirror in the analysis chip of the first embodiment.
[FIG. 5] A diagram illustrating an analysis chip of a second embodiment.
[FIG. 6] A diagram illustrating an analysis chip of a third embodiment.

### DESCRIPTION OF EMBODIMENTS

Embodiments of the present invention will be described in detail below with reference to the drawings.

In all the drawings, the same components are assigned the same reference numerals, and descriptions will not be repeated as appropriate.

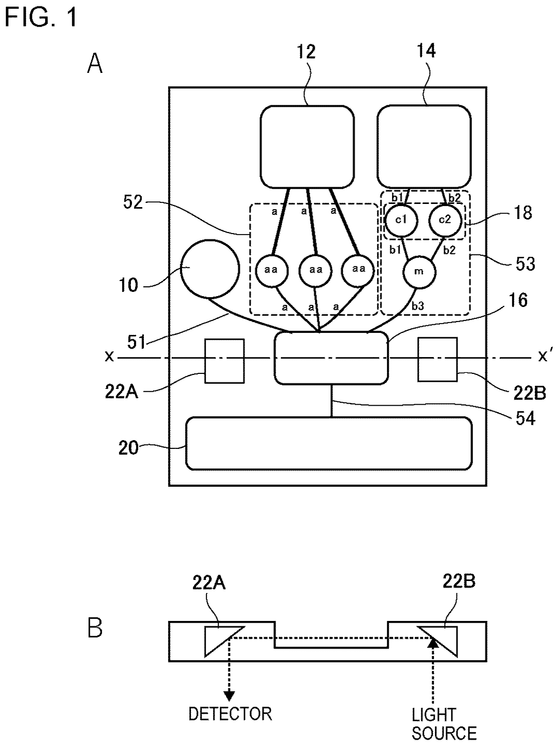

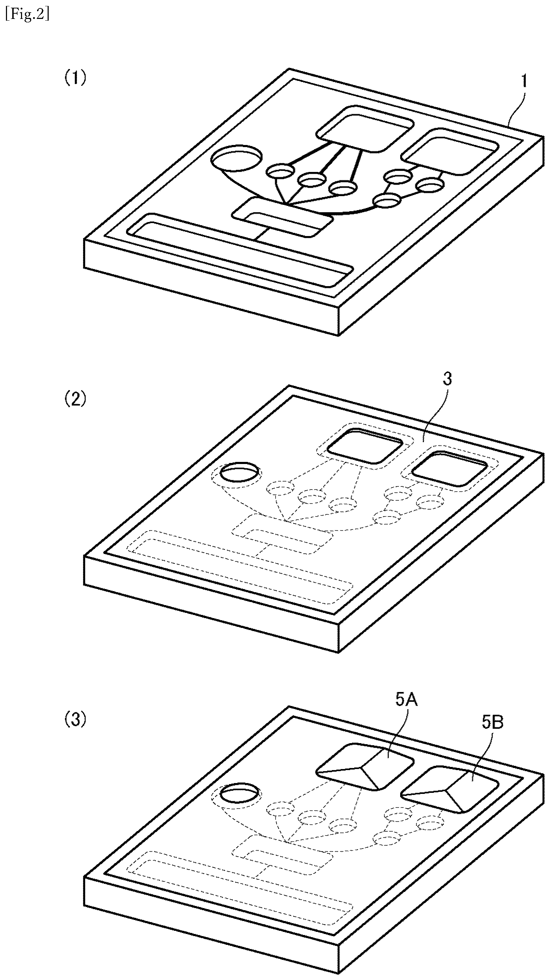

In order to avoid complexity, (i) when a plurality of identical components are present within the same drawing, only one of the components may be assigned a reference numeral, and reference numerals may not be assigned to all of the components, or (ii) especially in FIG. 2 and the subsequent figures, components that are the same as those in FIGS. 1A and 1B may not be assigned reference numerals again.

All the drawings are for illustration purposes only. The shapes, dimensional ratios, and the like of the respective members in the drawings do not necessarily correspond to those of actual articles.

In the present specification, the expression "X to Y" in the description of numerical ranges means X or greater and Y or less, unless otherwise specified. For example, "1% to 5% by mass" means "1% by mass or greater and 5% by mass or less".

Some abbreviations and technical terms used in the present specification are described below.
·NADH: Abbreviation for nicotinamide adenine dinucleotide.
·Thio-NAD: Abbreviation for thionicotinamide adenine dinucleotide.
· Thio-NADH: Abbreviation for thionicotinamide adenine dinucleotide, reduced form.
· Dehydrogenase: A general term for an enzyme that catalyzes a so-called dehydrogenation reaction, in which hydrogen is removed from a substrate and transferred to another acceptor. Specific examples thereof include 3α-hydroxysteroid dehydrogenase (3α-HSD).

Hereinafter, a first embodiment, a second embodiment, and a third embodiment, which are embodiments of the present invention, will be described. These allow sequential analysis steps, which have conventionally required specially trained personnel, to be performed with relatively simple operations such as applying a centrifugal force, by using a carefully designed analysis chip.

### <First Embodiment>

FIGS. 1A and 1B are diagrams schematically showing an analysis chip of a first embodiment.

This analysis chip can have, for example, the following configuration.

· A specimen introduction portion 10 (hereinafter, simply referred to as a "specimen introduction portion 10") into which a liquid specimen containing a protein or nucleic acid labeled with an enzyme-labeled antibody, or a liquid specimen containing an enzyme-labeled antibody and a protein or nucleic acid is introduced
· A washing solution storage portion 12
· A dissolving solution storage portion 14
· A reaction portion 16 (hereinafter, simply referred to as a "reaction portion 16") provided with an immobilized antibody or nucleic acid
· A first flow path 51 (hereinafter, simply referred to as a "first flow path 51") that connects the specimen introduction portion 10 and the reaction portion 16
· A second flow path 52 (hereinafter, simply referred to as a "second flow path 52") that connects the washing solution storage portion 12 and the reaction portion 16
· A third flow path 53 (hereinafter, simply referred to as a "third flow path 53") that connects the dissolving solution storage portion 14 and the reaction portion 16

A dry reagent enclosing portion 18 (hereinafter, simply referred to as a "dry reagent enclosing portion 18") in which a dry reagent containing NADH, a substrate for an enzyme in the above-described enzyme-labeled antibody, thio-NAD, and dehydrogenase is enclosed is provided midway in the third flow path 53.

In addition, this analysis chip preferably further has the following configuration.
· A waste liquid storage portion 20

The waste liquid storage portion 20 is connected to the reaction portion 16 through a fourth flow path 54.
· A mirror 22A and a mirror 22B

FIG. 1B is a cross-sectional view of the analysis chip shown in FIG. 1A taken along line xx'. As shown in FIG. 1B, the mirrors 22A and 22B are installed such that light from one surface side of the analysis chip is guided to the reaction portion 16 and the guided light is emitted to the outside of the analysis chip. With such a configuration, absorbance measurement, which will be described below, can be easily performed.

Additionally, the second flow path 52 preferably includes three to five sub flow paths a that connect the washing solution storage portion 12 and the reaction portion 16 (in FIG. 1A, three sub flow paths are shown). It is preferable that the sub flow paths a are different from each other in at least one of a cross-sectional area, a length, and hydrophilicity of a flow path inner wall.

A measurement portion aa of a washing solution can be provided midway in each of the sub flow paths. By providing the measurement portion aa, it is easier to sequentially feed a constant amount of the washing solution to the reaction portion 16.

Specifically, the sub flow paths (between the washing solution storage portion 12 and the measurement portions aa) located upstream of the measurement portions aa are designed such that the cross-sectional areas, the lengths, and the hydrophilicity of the flow path inner walls of the sub flow paths a are approximately the same, and the washing solution flows through the sub flow paths to the measurement portions aa in approximately the same amount of time when a predetermined centrifugal force is applied. On the other hand, the sub flow paths (between the measurement portions aa and the reaction portion 16) located downstream of the measurement portions aa are designed such that the sub flow paths a are different from each other in at least one of the cross-sectional area, the length, and the hydrophilicity of the flow path inner wall. In a case where the second flow path 52 is designed in this way, almost the same amount of the washing solution is first stored in the three measurement portions aa almost simultaneously, when a centrifugal force is applied to the analysis chip. After that, depending on the ease of flow of the sub flow paths located downstream of the measurement portions aa, the washing solution stored in each of the three measurement portions aa reaches the reaction portion 16 with a time delay, from each of the measurement portions aa.

In addition, the third flow path 53 preferably includes two sub flow paths, a sub flow path b1 and a sub flow path b2, extending from the dissolving solution storage portion 14, a mixing portion m to which the sub flow path b1 and the sub flow path b2 are connected, a sub flow path b3 that connects the mixing portion m to the reaction portion 16, a first dry reagent enclosing portion c1 provided midway in the sub flow path b1, and a second dry reagent enclosing portion c2 provided midway in the sub flow path b2.

Preferably, one of the first dry reagent enclosing portion c1 and the second dry reagent enclosing portion c2 encloses NADH and a substrate for the enzyme in the enzyme-labeled antibody (which is introduced into the specimen introduction portion 10), and the other thereof encloses thio-NAD and dehydrogenase.

From the viewpoint of miniaturization and the viewpoint of using amounts of a specimen, a reagent, a chemical liquid, and the like required for accurate analysis, the size of the analysis chip shown in FIG. 1A can be set to a length of 1 cm × a width of 1 cm to a length of 12 cm × a width of 12 cm.

Each of the above-described portions and each of the above-described flow paths are formed as recessed portions on a surface of a plate-like member 1, as shown in (1) of FIG. 2, as an example. As shown in (2) of FIG. 2, by covering the recessed portions of the plate-like member 1 with a lid member 3 from above and fixing the lid member 3, the recessed portions can stably store liquids or function as liquid flow paths (microchannel).

An opening portion (hole) can be provided in a part of the lid member 3. Specifically, the opening portion (hole) can be provided in a portion corresponding to the specimen introduction portion 10, a portion corresponding to the washing solution storage portion 12, and a portion corresponding to the dissolving solution storage portion 14.

Additionally, as necessary, the waste liquid storage portion 20 can be provided with an auxiliary flow path for pressure adjustment, and an opening portion (hole) can also be provided at the end of the auxiliary flow path.

As shown in (3) of FIG. 2, a washing solution package 5A in which the washing solution is enclosed with a first packaging material can be provided over the opening portion of the portion corresponding to the washing solution storage portion 12. With such a configuration, when the first packaging material of the washing solution package 5A is destroyed, the washing solution is supplied to the washing solution storage portion 12.

In addition, a dissolving solution package 5B in which the dissolving solution is enclosed with a second packaging material can be provided over the opening portion of the portion corresponding to the dissolving solution storage portion 14. With such a configuration, when the second packaging material of the dissolving solution package 5B is destroyed, the dissolving solution is supplied to the dissolving solution storage portion 14.

In the first embodiment, the first flow path 51, the second flow path 52, and the third flow path 53 are different from each other in at least one of the cross-sectional area and the hydrophilicity of the flow path inner wall.

As an example, the following is possible: the cross-sectional area of the first flow path 51 > the cross-sectional area of the second flow path 52 > the cross-sectional area of the third flow path 53.

More specifically, the following can be set: the cross-sectional area of the flow path listed in (1) below > the cross-sectional area of the flow path listed in (2) > the cross-sectional area of the flow path listed in (3). It is preferable that the cross-sectional areas of the flow paths listed in (1) are substantially the same, and it is preferable that the cross-sectional areas of the flow paths listed in (2) are substantially the same.
(1) The cross-sectional area of the first flow path 51, the cross-sectional areas of the sub flow paths a located upstream of the measurement portions aa, the cross-sectional area of the sub flow path b1 located upstream of the first dry reagent enclosing portion c1, and the cross-sectional area of the sub flow path b2 located upstream of the second dry reagent enclosing portion c2
(2) The cross-sectional areas of the sub flow paths a located downstream of the measurement portions aa, the cross-sectional area of the sub flow path b1 located downstream of the first dry reagent enclosing portion c1, and the cross-sectional area of the sub flow path b2 located downstream of the second dry reagent enclosing portion c2
(3) The cross-sectional area of the sub flow path b3

These cross-sectional areas of the flow paths are typically within a range of 1 to 25 mm². In a case where the cross-sectional area within a single flow path varies depending on the location, a minimum value of the cross-sectional area within the flow path can be regarded as the cross-sectional area of the flow path.

As another specific example, the following is possible: the hydrophilicity of the inner wall of the first flow path 51 > the hydrophilicity of the inner wall of the second flow path 52 > the hydrophilicity of the inner wall of the third flow path 53.

More specifically, the following can be set: the hydrophilicity of the flow path listed in (1) below > the hydrophilicity of the flow path listed in (2) > the hydrophilicity of the flow path listed in (3). It is preferable that the hydrophilicity of the flow paths listed in (1) is substantially the same, and it is preferable that the hydrophilicity of the flow paths listed in (2) is substantially the same.
(1) The hydrophilicity of the first flow path 51, the hydrophilicity of the sub flow paths a located upstream of the measurement portions aa, the hydrophilicity of the sub flow path b1 located upstream of the first dry reagent enclosing portion c1, and the hydrophilicity of the sub flow path b2 located upstream of the second dry reagent enclosing portion c2
(2) The hydrophilicity of the sub flow paths a located downstream of the measurement portions aa, the hydrophilicity of the sub flow path b1 located downstream of the first dry reagent enclosing portion c1, and the hydrophilicity of the sub flow path b2 located downstream of the second dry reagent enclosing portion c2
(3) The hydrophilicity of the sub flow path b3

The hydrophilicity can be indicated by, for example, a contact angle of water.

Additionally, the flow paths may be different from each other in both the cross-sectional area and the hydrophilicity.

With such a configuration described above, the ease of flow of the fluid in each of the first flow path 51, the second flow path 52, and the third flow path 53 varies. Therefore, when a predetermined centrifugal force is applied to the analysis chip, the specimen, the washing solution, and the dissolving solution can flow sequentially.

Preferably, the magnitude of the predetermined centrifugal force increases at least once, and more preferably twice or more, as time elapses. The increase in centrifugal force may be continuous or discontinuous. In addition, the centrifugal force that has once increased may decrease as long as the specimen, the washing solution, and the dissolving solution flow appropriately.

The direction of the centrifugal force is typically a downward direction in FIG. 1A. However, the direction of the centrifugal force need not be strictly the downward direction in FIG. 1A as long as the specimen, the washing solution, and the dissolving solution flow through the flow paths.

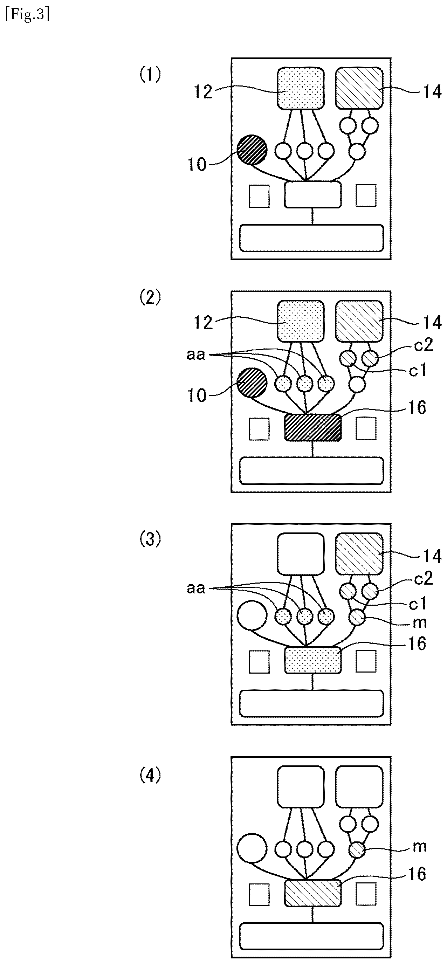

The flow of the specimen, the washing solution, and the dissolving solution when a predetermined centrifugal force is applied to the analysis chip will be described more specifically with reference to (1) to (4) of FIG. 3.

In (1) of FIG. 3, a state before the centrifugal force is applied is shown in which the specimen is introduced into the specimen introduction portion 10, the washing solution is introduced into the washing solution storage portion 12, and the dissolving solution is introduced into the dissolving solution storage portion 14.

As an example, the specimen can be a protein or nucleic acid that has been labeled with an enzyme-labeled antibody in advance. As another example, an enzyme-labeled antibody and a liquid specimen containing a protein or nucleic acid may be each introduced into the specimen introduction portion 10, and the specimen may be labeled in the specimen introduction portion 10.

The introduction of the washing solution can be performed, for example, by destroying the first packaging material of the washing solution package 5A shown in (3) of FIG. 2. Similarly, the introduction of the dissolving solution can be performed, for example, by destroying the second packaging material of the dissolving solution package 5B shown in (3) of FIG. 2. The introduction of the washing solution or the introduction of the dissolving solution may be performed by using a dropper, a pipette, or the like without using the washing solution package 5A and the dissolving solution package 5B.

In (2) of FIG. 3, a state is shown in which, by applying a relatively small first centrifugal force to the analysis chip, at least the liquid specimen introduced into the specimen introduction portion 10 flows inside the first flow path 51 and flows to the reaction portion 16. The direction of the centrifugal force is typically a downward direction in the figure. However, the direction of the centrifugal force need not be strictly the downward direction in the figure as long as the specimen flows inside the first flow path 51 (the same applies to the direction of the centrifugal force in (3) and (4) of FIG. 3).

In addition, in (2) of FIG. 3, the washing solution introduced into the washing solution storage portion 12 reaches the measurement portion aa provided midway in the second flow path 52 and is stored in the measurement portion aa.

Further, in (2) of FIG. 3, the dissolving solution introduced into the dissolving solution storage portion 14 reaches the dry reagent enclosing portion 18 (the first dry reagent enclosing portion c1 and the second dry reagent enclosing portion c2) provided midway in the third flow path 53. Then, the dry reagent is dissolved in the dissolving solution.

In (3) of FIG. 3, a state is shown in which, after substantially all (or a necessary amount) of the liquid specimen introduced into the specimen introduction portion 10 has reached the reaction portion 16, a second centrifugal force greater than the first centrifugal force is applied to the analysis chip, and the washing solution introduced into the washing solution storage portion 12 (more specifically, the washing solution introduced into the washing solution storage portion 12 and then stored in the measurement portion aa) flows to the reaction portion 16.

Incidentally, in this case, the liquid in the dry reagent enclosing portion 18 (the first dry reagent enclosing portion c1 and the second dry reagent enclosing portion c2), that is, the solution obtained by dissolving the dry reagent, may move to the mixing portion m. However, this movement is not essential, and the solution obtained by dissolving the dry reagent mixed in (4) of FIG. 3 need only flow to the reaction portion 16.

Additionally, in this case, the liquid specimen in the reaction portion 16 typically moves to the waste liquid storage portion 20 (not shown in (3) of FIG. 3).

In (4) of FIG. 3, a state is shown in which, after substantially all of the washing solution introduced into the measurement portion aa has reached the reaction portion 16, a third centrifugal force greater than the second centrifugal force is applied to the analysis chip, and the solution (mixed solution) in which the dry reagent has been dissolved in the dissolving solution introduced into the dissolving solution storage portion 14 flows from the mixing portion m to the reaction portion 16. That is, in (4) of FIG. 3, the dry reagent in the dry reagent enclosing portion 18 is dissolved in the dissolving solution, and the resulting solution (containing NADH, a substrate for the enzyme in the enzyme-labeled antibody, thio-NAD, and dehydrogenase) flows into the reaction portion 16.

By the sequential movement of each liquid due to a centrifugal force as described above:
(i) first, the specimen reaches the reaction portion 16, and the specimen reacts (binds) with an antibody or nucleic acid immobilized in the reaction portion 16;
(ii) next, an unreacted specimen and some or all of the components that may interfere with subsequent analysis are discharged from the reaction portion 16 by the washing solution flowing from the washing solution storage portion 12; and
(iii) after that, the solution obtained by dissolving the dry reagent is introduced into the reaction portion 16, thereby allowing a color development reaction to proceed.

The enzyme reaction itself in (iii) above is described in Patent Documents 1 and 2. Briefly, in a case where a protein or nucleic acid to be measured is contained in the specimen, thio-NAD is converted into thio-NADH by dehydrogenase.

Thio-NADH has a property of absorbing light having a wavelength of 405 nm. Therefore, by measuring the absorbance of the liquid stored in the reaction portion 16 using light at a wavelength of 405 nm, it is possible to confirm the presence or absence of the protein or nucleic acid to be measured in the specimen or to perform quantification.

Incidentally, in order to accelerate the progress of the color development reaction, at least the portion of the reaction portion 16 in the analysis chip may be heated in (iii) above. The heating temperature can be, for example, 35°C to 40°C.

Specifically, as shown in FIG. 1B, the absorbance of the liquid stored in the reaction portion 16 can be measured by using a light source and a detector. In this manner, the amount (concentration) of thio-NADH generated by the progress of the color development reaction can be quantified as the absorbance.

As described above, the specific protein or nucleic acid in the specimen can be quantified or the presence or absence thereof can be confirmed.

That is, by preparing the analysis chip as shown in FIGS. 1A, 1B, and 2, the following steps are performed:
· a step of introducing the liquid specimen containing a protein or nucleic acid labeled with an enzyme-labeled antibody into the specimen introduction portion of the analysis chip;
· a step of applying a centrifugal force to the analysis chip to: (1) first cause the specimen to reach the reaction portion 16; after the specimen reaches the reaction portion 16, (2) cause the washing solution introduced into the washing solution storage portion 12 to reach the reaction portion 16 and discharge the washing solution from the reaction portion 16; and then (3) cause the solution, in which the dry reagent has been dissolved in the dissolving solution introduced into the dissolving solution storage portion 14, to reach the reaction portion 16; and
· a step of measuring the absorbance of the liquid stored in the reaction portion 16,
thereby enabling rapid and simple analysis of the specific protein or nucleic acid in the specimen at the point of sample collection, without the need for operations such as dispensing a reaction test solution into a microplate.

For reference, <Analysis Protocol (Reference)> below describes that a specific protein or nucleic acid in the specimen can be analyzed by such sequential processing described above.

It can also be said that the analysis method for the specimen using the analysis chip described in the present specification is a method in which the protocol described in <Analysis Protocol (Reference)> below can be performed within a single analysis chip.

Various matters will be described in more detail below.

### · Plate-like Member 1 and Lid Member 3

The materials of the plate-like member 1 and the lid member 3 are typically resin. The plate-like members 1 and 3 can be made of any resin as long as it does not alter the stored or flowing liquids and is not degraded by any of the liquids. In consideration of the use record in the field of life science, the chemical resistance, the ability to immobilize antibodies or nucleic acids, and the like, the material of the plate-like member 1 is preferably polystyrene, polypropylene, polycarbonate, or the like.

The material of the lid member 3 is preferably polyester such as polyethylene terephthalate or polyolefin such as polypropylene.

The plate-like member 1 can be manufactured by an injection molding method using a thermoplastic resin (preferably the above-mentioned polystyrene or the like) as a raw material. That is, by preparing a mold including protruding portions corresponding to the recessed portions as shown in (1) of FIG. 2 and injecting the molten thermoplastic resin into the mold, the plate-like member 1 can be manufactured.

In a case where the hydrophilicity of the respective flow paths is to be varied, for example, at least some of the flow paths in the plate-like member 1 are subjected to a hydrophilic treatment or a hydrophobic treatment. In this case, as necessary, a portion where the hydrophilic treatment or the hydrophobic treatment is not desired is masked.

The hydrophilic treatment can be performed, for example, by bringing a polymer having a hydrophilic group into contact with the portion to be hydrophilized and allowing the polymer to adhere.

Examples of the hydrophobic treatment include a hexamethyldisilazane (HMDS) treatment. The HMDS treatment is commonly used in semiconductor wafer processing. Additionally, the hydrophobic treatment can also be performed by using a silane coupling agent. By using a silane coupling agent, a hydrophilic group such as an OH group present on the surface of the plate-like member 1 can be substituted with a hydrophobic group.

Further, by means such as forming fine irregularities on the inner walls of the flow paths or adjusting the roughness of the inner walls of the flow paths, it is also possible to change the hydrophilicity of each flow path.

The form of the lid member 3 is not particularly limited as long as there is no leakage of liquid from a portion other than the portion having the opening portion (hole) when the lid member 3 is covered and fixed onto the plate-like member 1. The lid member 3 can be in the form of a film, a sheet, a thin plate, or the like.

### · Washing Solution Package 5A and Washing Solution

The first packaging material constituting the washing solution package 5A can stably hold the liquid inside when no external force is applied, but it is preferable for the material to be a material that can be broken when pressed with an appropriate force or a material that is smoothly pierced when punctured with a needle. Since such packaging materials have been also employed in conventional analysis chips or test kits, for example, "Fujifilm Dry-Chem IMMUNO AG Handy COVID-19 Ag" manufactured by FUJIFILM Medical Co., Ltd., the technology can be employed for use as appropriate.

The washing solution contained in the washing solution package 5A is preferably a buffer solution containing a surfactant. By using such a washing solution, it is possible to more efficiently discharge the specimen or impurities remaining in the reaction portion 16, which did not bind to the antibody or nucleic acid immobilized in the reaction portion 16, from the reaction portion 16, thereby moving the specimen or impurities to the waste liquid storage portion 20.

As the surfactant, a surfactant that has been conventionally used for the analysis of proteins or nucleic acids can be used without any particular limitation. Preferred examples thereof include a nonionic surfactant, and more preferred examples thereof include Tween-type surfactants such as Tween 20 and Tween 80, Triton X-100, and NP-40.

As the buffer solution, tris-buffered saline (TBS) is preferably used in terms of compatibility with the specimen.

Of course, the types of surfactants and buffer solutions are not limited as long as appropriate analysis can be performed.

### · Dissolving Solution Package 5B and Dissolving Solution

The second packaging material constituting the dissolving solution package 5B can be the same as the above-mentioned first packaging material.

The dissolving solution contained in the dissolving solution package 5B is preferably a buffer solution.

As the buffer solution, a Tris-HCl buffer is preferably used. Of course, the types of surfactants and buffer solutions are not limited as long as appropriate analysis can be performed.

### · Regarding Specimen

The specimen to be introduced into the specimen introduction portion 10 is in a liquid form and is not particularly limited as long as it can contain a protein or nucleic acid to be measured. The specimen may be a biological tissue, a body fluid, or the like.

The specimen may be labeled with an enzyme-labeled antibody before being introduced into the specimen introduction portion 10 or may not be labeled. In the latter case, both the specimen and the enzyme-labeled antibody are introduced into the specimen introduction portion 10.

The enzyme-labeled antibody is preferably a secondary antibody labeled with alkaline phosphatase (ALP).

### · Specimen Introduction Portion 10

The specimen introduction portion 10 is not particularly limited as long as it has a volume capable of storing a specimen amount necessary for the analysis.

The volume of the specimen introduction portion 10 is, for example, 1 to 300 µL, with the volume depending on the overall size of the entire analysis chip.

### · Washing Solution Storage Portion 12

The washing solution storage portion 12 is not particularly limited as long as it has a volume necessary for storing the washing solution to be supplied from the washing solution package 5A.

The volume of the washing solution storage portion 12 is, for example, 3 to 3,000 µL, with the volume depending on the overall size of the entire analysis chip.

### · Dissolving Solution Storage Portion 14

The dissolving solution storage portion 14 is not particularly limited as long as it has a volume necessary for storing the washing solution to be supplied from the dissolving solution package 5B.

The volume of the dissolving solution storage portion 14 is, for example, 1 to 3,000 µL, with the volume depending on the overall size of the entire analysis chip.

### · Reaction Portion 16

The reaction portion 16 is provided with an immobilized antibody or nucleic acid.

As an example, an antibody or nucleic acid is immobilized to at least a part of the inner wall of the reaction portion 16. Before the plate-like member 1 is covered with the lid member 3 in manufacturing the analysis chip,
the antibody or the nucleic acid can be immobilized onto the portion of the plate-like member 1 that corresponds to the reaction portion 16. As a method of immobilization, a known method can be applied as appropriate. For example, the same method as the method of immobilizing an antibody or nucleic acid onto a microplate in a laboratory can be employed. As a specific example, there is a method in which sections 1 and 2 described in <Analysis Protocol (Reference)> below are performed on the portion of the plate-like member 1 that corresponds to the reaction portion 16.

As another example, the reaction portion 16 may include particles (beads or the like) to which the antibody or nucleic acid is immobilized in the reaction portion 16, thereby providing the immobilized antibody or nucleic acid. In a case where these particles do not hinder the movement of the liquid from the reaction portion 16 to the waste liquid storage portion 20 and do not interfere with the absorbance measurement of the liquid in the reaction portion 16, this example is easier to manufacture and leads to cost savings.

The antibody or nucleic acid to be immobilized need only be selected as appropriate according to the type of the protein or nucleic acid to be analyzed. That is, an antibody or nucleic acid that can specifically bind to the protein or nucleic acid to be analyzed is immobilized in advance in the reaction portion 16.

The volume of the reaction portion 16 is, for example, 1 to 300 µL, with the volume depending on the overall size of the entire analysis chip.

### · First Flow Path 51

The first flow path 51 is not particularly limited as long as the liquid specimen introduced into the specimen introduction portion 10 can be fed from the specimen introduction portion 10 to the reaction portion 16.

### · Second Flow Path 52 and Sub Flow Paths

The second flow path 52 is not particularly limited as long as the washing solution stored in the washing solution storage portion 12 can be fed from the washing solution storage portion 12 to the reaction portion 16.

As mentioned above, the second flow path 52 preferably includes three to five sub flow paths a that connect the washing solution storage portion 12 and the reaction portion 16 (in FIG. 1A, three sub flow paths are shown). It is preferable that the sub flow paths a are different from each other in at least one of the cross-sectional area, the length, and the hydrophilicity of the flow path inner wall.

In addition, the measurement portion aa of the washing solution can be provided midway in each of the sub flow paths. By providing the measurement portion aa, it is possible to feed a constant amount of the washing solution to the reaction portion 16. The volume of one measurement portion aa is, for example, 1 to 300 µL, with the volume depending on the overall size of the entire analysis chip.

The expression "the sub flow paths a being different from each other in at least one of the cross-sectional area, the length, and the hydrophilicity of the flow path inner wall" means that the ease with which the washing solution flows differs among the sub flow paths a on the left side, the center, and the right side in FIG. 1A. Therefore, in each of the sub flow paths a, the time it takes for the washing solution to flow from the washing solution storage portion 12 to the reaction portion 16 varies. Alternatively, in each of the sub flow paths a, the time it takes for the washing solution to flow from the measurement portion aa to the reaction portion 16 varies.

By appropriately adjusting at least one of the cross-sectional area, the length, and the hydrophilicity of the flow path inner wall of each of the sub flow paths a, it is possible to sequentially move the washing solution, for example as described below, when a predetermined centrifugal force is applied or when the centrifugal force increases over time.
(i) Substantially all of the washing solution that has flowed from one measurement portion aa and has reached the reaction portion 16 is discharged to the waste liquid storage portion 20; after the discharge is completed, (ii) the washing solution that has flowed from the second measurement portion aa reaches the reaction portion 16, and substantially all of the reached washing solution is discharged to the waste liquid storage portion 20; and after the discharge is completed, (iii) the washing solution that has flowed from the third measurement portion aa reaches the reaction portion 16, and substantially all of the reached washing solution is discharged to the waste liquid storage portion 20.

Incidentally, from the viewpoint of ensuring the measurement by the measurement portion aa, it is preferable that the volume of the measurement portion aa (the total volume in a case where a plurality of measurement portions aa are provided) is substantially the same as or greater than the total volume of the washing solution flowing out from the washing solution storage portion 12. That is, it is preferable to design the volume of the measurement portion aa to be properly large such that all of the washing solution flowing out from the washing solution storage portion 12 can be stored.

It is preferable to repeat the operation of once discharging substantially all of the washing solution introduced into the reaction portion 16 from the reaction portion 16 and then introducing a new washing solution into the reaction portion 16, as described above, as an unreacted specimen or a component that may interfere with the analysis is more easily removed. Such a method of supplying the washing solution is more effective in washing than continuously supplying the washing solution to the reaction portion 16 without interruption.

(It may be helpful to recall that, in laundry "rinsing", repeating rinsing and spinning is more effective at removing detergent with less water than continuously pouring water into the washing tub.)

Incidentally, including three to five sub flow paths a means that the reaction portion 16 undergoes washing three to five times.

In the high-sensitivity ELISA methods in Patent Documents 1 and 2, the microplate was washed about 10 times before the enzyme reaction. This is because, particularly in high-sensitivity measurement, it was considered that an antibody that did not react (bind) with the antibody or nucleic acid immobilized onto the microplate may hinder appropriate measurement.

However, it is unrealistic to incorporate a mechanism for performing washing and discharge of the washing solution 10 times in an analysis chip that needs to be miniaturized. Therefore, the present inventor has newly discovered that, through optimization of the reaction protocol and the like, sufficiently high-sensitivity measurement can be performed even with only three to five washings of the reaction portion 16. This is supported by the fact that appropriate measurement can be performed by "washing the microplate three times" in item (3) of section 3 of <Analysis Protocol (Reference)> below.

### · Third Flow Path 53, Sub Flow Paths, and Dry Reagents

The third flow path 53 is not particularly limited as long as the dissolving solution stored in the dissolving solution storage portion 14 can be fed to the reaction portion 16 through the dry reagent enclosing portion 18.

As mentioned above, the third flow path 53 preferably includes two sub flow paths, the sub flow path b1 and the sub flow path b2, extending from the dissolving solution storage portion 14, the mixing portion m to which the sub flow path b1 and the sub flow path b2 are connected, the sub flow path b3 that connects the mixing portion m to the reaction portion 16, the first dry reagent enclosing portion c1 provided midway in the sub flow path b1, and the second dry reagent enclosing portion c2 provided midway in the sub flow path b2.

Preferably, one of the first dry reagent enclosing portion c1 and the second dry reagent enclosing portion c2 encloses NADH and a substrate for the enzyme in the enzyme-labeled antibody (which is introduced into the specimen introduction portion 10), and the other thereof encloses thio-NAD and dehydrogenase.

The volume of the mixing portion m is, for example, 1 to 300 µL, the volume of the first dry reagent enclosing portion c1 is, for example, 0.5 to 150 µL, and the volume of the second dry reagent enclosing portion c2 is, for example, 0.5 to 150 µL, with all of these volumes depending on the overall size of the analysis chip.

As described above, by configuring the third flow path 53 with two or more sub flow paths and separately disposing the dry reagent containing "NADH and a substrate for the enzyme in the enzyme-labeled antibody" and the dry reagent containing "thio-NAD and dehydrogenase" midway in the sub flow paths, it is possible to suppress the reaction between the reagents before the analysis chip is used (before specimen analysis). By using the dry reagent, the usable period of the analysis chip can be further extended.

In the analysis chip shown in FIG. 1A, two sub flow paths, the sub flow path b1 and the sub flow path b2, extend from a "single" dissolving solution storage portion 14, and the first dry reagent enclosing portion c1 is provided midway in the sub flow path b1, and the second dry reagent enclosing portion c2 is provided midway in the sub flow path b2. In such a configuration, the dissolving solution for the dry reagent (hereinafter, also referred to as a "first dry reagent") in the first dry reagent enclosing portion c1 and the dissolving solution for the dry reagent (hereinafter, also referred to as a "second dry reagent") in the second dry reagent enclosing portion c2 are the same.

Conventionally, it has been common sense to dissolve dry reagents used for specimen analysis or other biochemical experiments and analyses in the same dissolving solution as a dissolving solution (typically, a buffer solution) contained in a liquid material which is a raw material of the dry reagent. According to the first embodiment, in a case where the first dry reagent is a reagent obtained by freeze-drying a liquid material adjusted to a pH of about 9 with a buffer solution, the first dry reagent is dissolved with a buffer solution having a pH of about 9, and in a case where the second dry reagent is a reagent obtained by freeze-drying a liquid material adjusted to a pH of about 7 with a buffer solution, the second dry reagent is dissolved with a buffer solution having a pH of about 7. This is the conventional method of dissolving dry reagents based on common sense. In order to achieve such a dissolution method with the analysis chip, it is necessary to provide two dissolving solution storage portions 14 within a single analysis chip.

However, providing two dissolving solution storage portions 14 within the single analysis chip is not desirable in terms of the complexity of the analysis chip and an increase in associated cost. Therefore, the present inventor has challenged the above-described conventional wisdom. The present inventor has found that specimen analysis can be performed without any particular problem even when the dissolving solution for the first dry reagent and the dissolving solution for the second dry reagent are the same. This is supported by the fact that, in section 4 of <Analysis Protocol (Reference)> below, good analysis was performed even though the freeze-dried products R-1 and R-2 were dissolved in the same Tris-HCl buffer (pH 9.5).

The first dry reagent and the second dry reagent can be manufactured by using a known freeze-drying technology.

One of the first dry reagent and the second dry reagent can be manufactured by freeze-drying the liquid material (preferably having a pH of 8.5 to 10.5) containing NADH, a substrate for the enzyme in the enzyme-labeled antibody, and a buffer solution.

Similarly, the other of the first dry reagent and the second dry reagent can be manufactured by freeze-drying a liquid material (preferably having a pH of 6.5 to 8.5) containing thio-NAD, dehydrogenase, and a buffer solution.

The enclosed amount of the dry reagent containing NADH and the substrate for the enzyme in the enzyme-labeled antibody, and the enclosed amount of the dry reagent containing thio-NAD and dehydrogenase are not particularly limited. These amounts need only be determined as appropriate according to the overall size of the analysis chip, the volumes of the first dry reagent enclosing portion c1 and the second dry reagent enclosing portion c2, the planned amount of the specimen to be introduced, the required measurement sensitivity and accuracy, and the like.

### · Waste Liquid Storage Portion 20

The waste liquid storage portion 20 is not particularly limited as long as it has a volume necessary for storing various liquids to be supplied from the reaction portion 16.

The volume of the waste liquid storage portion 20 is, for example, 6 to 3,000 µL, with the volume depending on the overall size of the analysis chip.

### · Mirror 22A, Mirror 22B, and Surroundings Thereof (Including Modification Example)

From the viewpoint of improving analysis sensitivity, in absorbance measurement, it is preferable for light to pass through the liquid stored in the reaction portion 16 over as long a distance as possible. Specifically, as shown in FIG. 1B, it is preferable to dispose the mirror 22A and the mirror 22B so that light passes through the liquid stored in the reaction portion 16 over a distance equal to the length of the long side of the reaction portion 16, which is substantially rectangular when viewed from above, from the viewpoint of improving detection sensitivity and improving the quantitativeness of measurement.

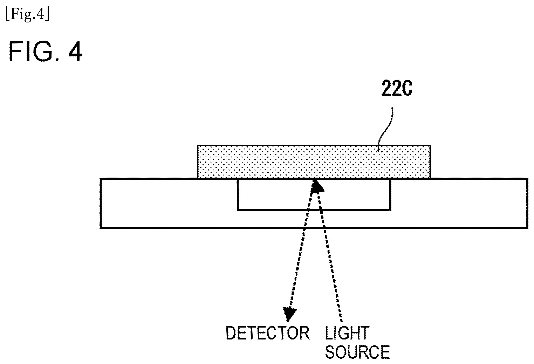

Meanwhile, as shown in FIG. 4, the analysis chip may be provided with only a single mirror 22C. In this case, measurement light is obliquely applied from a surface of the analysis chip opposite to a surface on which the mirror 22C is provided, and the reflected light from the mirror 22C is detected by the detector. In this case, the distance that light passes through the liquid stored in the reaction portion 16 tends to be shorter, and in terms of detection sensitivity and measurement quantitativeness, it tends to be inferior to the aspect shown in FIG. 1B. However, instead, it is possible to benefit from reduced manufacturing costs due to the simplification of the chip structure.

### · Analysis System for Specimen

An analysis system for a specimen can be configured by using:
the above-mentioned analysis chip;
a centrifugal force applying unit capable of applying a centrifugal force to the above-mentioned analysis chip; and
an absorbance measuring unit capable of measuring an absorbance of a liquid stored in the reaction portion 16 in the above-mentioned analysis chip.

Additionally, the analysis system for a specimen preferably includes a unit for applying an external force to the washing solution package 5A and the dissolving solution package 5B in the above-mentioned analysis chip to supply the washing solution in the washing solution package 5A to the washing solution storage portion 12 and to supply the dissolving solution in the dissolving solution package 5B to the dissolving solution storage portion 14. Alternatively, by destroying the washing solution package 5A and the dissolving solution package 5B, for example, by pressing each package with fingers, the washing solution may be introduced into the washing solution storage portion 12, and the dissolving solution may be introduced into the dissolving solution storage portion 14.

A specific form of the centrifugal force applying unit is not particularly limited. Any form and mechanism can be employed as long as a centrifugal force can be applied to the analysis chip. A centrifugal force of a predetermined magnitude need only be stably applied to the analysis chip after the analysis chip has been appropriately held or fixed. One centrifugal force applying unit may apply a centrifugal force to only one analysis chip or may apply a centrifugal force to a plurality of analysis chips simultaneously. By employing the centrifugal force applying unit as in the latter case, an advantage is obtained in that a plurality of specimens can be collectively analyzed at once, or a plurality of types of proteins or nucleic acids contained in one specimen can be collectively analyzed at once.

For the centrifugal force applying unit, for example, it is conceivable to apply technologies of tabletop or compact centrifuges or to apply technologies of CD players.

In the first embodiment, it is preferable to use a centrifugal force applying unit in which the magnitude (rotation speed) of the centrifugal force is variable. More preferably, the centrifugal force applying unit is preferably capable of changing the rotation speed (increasing the rotation speed) as time elapses based on the prior setting. That is, the centrifugal force applying unit is preferably configured to change the rotation speed continuously or discontinuously (increase the rotation speed) such that the first centrifugal force, the second centrifugal force, and the third centrifugal force mentioned above are sequentially applied to the chip as time elapses based on the prior setting (program).

The absorbance measuring unit is not particularly limited. Any unit capable of measuring the absorbance of the liquid stored in the reaction portion 16 can be employed. The absorbance measuring unit typically includes a light source and a detector capable of measuring the intensity of light. Since thio-NADH absorbs light having a wavelength of 405 nm, it is preferable that the light source emits at least light having a wavelength of 405 nm, and it is preferable that the detector can measure the intensity of at least light having a wavelength of 405 nm. From the viewpoint of miniaturization of the light source system or the like, a light emitting diode, a laser diode, or the like can be used as the light source.

The centrifugal force applying unit and the absorbance measuring unit may be integrated as a device, or the centrifugal force applying unit and the absorbance measuring unit may be separate devices.

### <Second Embodiment>

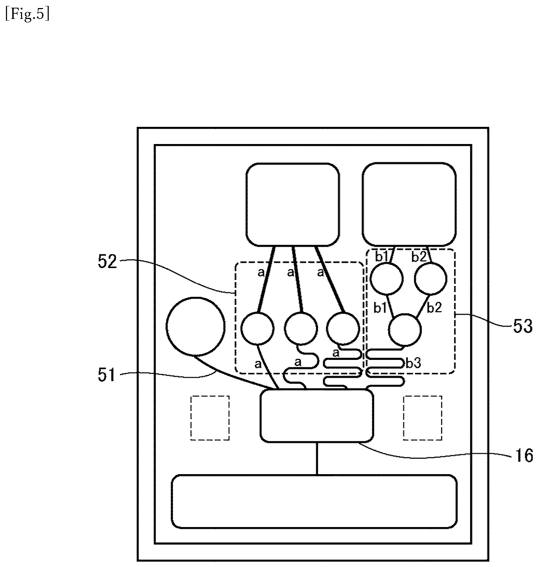

FIG. 5 is a diagram schematically showing an analysis chip of a second embodiment.

Many parts of the analysis chip of the second embodiment are common to the analysis chip of the first embodiment. Therefore, the description of the common parts will not be repeated basically. In other words, matters not described below can be referred to in the contents described in the first embodiment.

In the analysis chip of the second embodiment, the first flow path 51, the second flow path 52, and the third flow path 53 are different from each other in at least the length (flow path length). In this respect, the analysis chip of the second embodiment is different from the analysis chip of the first embodiment. In the analysis chip of the second embodiment, the flow path length of the first flow path 51 < the flow path length of the second flow path 52 < the flow path length of the third flow path 53 is typically satisfied. In FIG. 5, the flow path lengths of the first flow path 51, the second flow path 52, and the third flow path 53 are varied by partially meandering the flow paths.

In a case where each flow path includes a plurality of sub flow paths, it is preferable that all the sub flow paths satisfy the relationship of the above-described inequality. For example, in a case where the second flow path includes three sub flow paths a as shown in FIG. 5, and the flow path length of the sub flow path a on the left side in the figure < the flow path length of the sub flow path a at the center < the flow path length of the sub flow path a on the right side is satisfied, the flow path length of the sub flow path a on the left side is greater than the flow path length of the first flow path, and the flow path length of the sub flow path a on the right side is smaller than the flow path length of the third flow path.

(The third flow path also includes the sub flow paths, and it is preferable that a shorter flow path length of the flow path indicated by reference numeral b1-b1-b3 and the flow path indicated by reference numeral b2-b2-b3 is shorter than the flow path length of the sub flow path a on the right side.)

By making the flow paths different from each other in at least the length, even in a case where the cross-sectional areas of the flow paths are substantially the same and a centrifugal force whose magnitude is substantially constant over time is applied to the analysis chip, sequential movement of the liquids, particularly the introduction and discharge of each liquid into and from the reaction portion 16, can be performed at appropriate timings, as described in the first embodiment. As a result, it is possible to easily and rapidly analyze the protein or nucleic acid in the specimen.

It is preferable that the magnitude of the centrifugal force is substantially constant over time, but it is not excluded that the magnitude of the centrifugal force changes over time. By integrally considering and designing the flow path length of each flow path and the magnitude of the centrifugal force, it is possible to optimize, in particular, the timings of introduction and discharge of each liquid into and from the reaction portion 16.

Even in a case where the cross-sectional areas and the hydrophilicity of the inner walls of the respective flow paths are substantially the same, by making the flow path lengths of the respective flow paths different, it is possible to control the timings of introduction and discharge of each liquid into and from the reaction portion 16. However, in addition to varying the flow path lengths of the respective flow paths, by also making the cross-sectional areas and/or the hydrophilicity of the inner walls of the respective flow paths different, the ease of flow of the fluid in each flow path may be adjusted.

### <Third Embodiment>

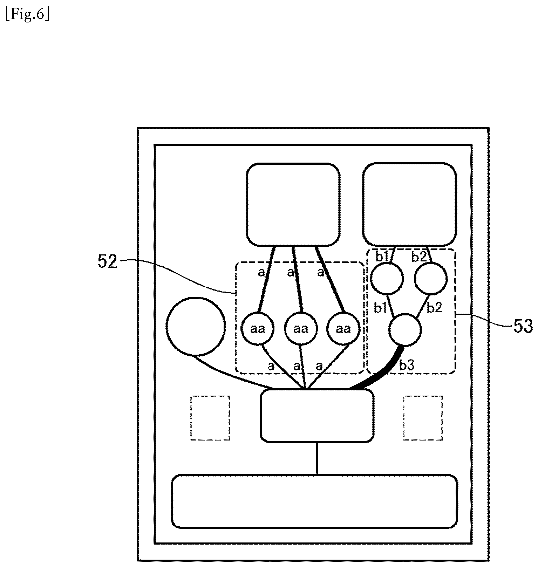

FIG. 6 is a diagram schematically showing an analysis chip of a third embodiment.

Many parts of the analysis chip of the third embodiment are common to the analysis chip of the first embodiment. Therefore, the description of the common parts will not be repeated basically. In other words, matters not described below can be referred to in the contents described in the first embodiment.

In the analysis chip of the third embodiment, a blocking portion is provided midway in either the second flow path 52 or the third flow path 53, and the blocking portion prevents the liquid from passing through. In the analysis chip shown in FIG. 6, at least a part of the sub flow path b3 in the third flow path 53 is blocked, thereby preventing the liquid from passing through.

The blocking portion can be destroyed by light or heat. The blocking portion is destroyed by light or heat (the blocking portion is opened), thereby allowing the liquid to pass through. In FIG. 6, the sub flow path b3 is unblocked, thereby allowing the liquid (the solution obtained by dissolving the dry reagent) to pass through the sub flow path b3.

By preparing the analysis chip including the blocking portion as shown in FIG. 6 and performing each of the following steps, preferably in the order described herein, the specimen can be analyzed.
· A step of introducing a liquid specimen containing a protein or nucleic acid labeled with an enzyme-labeled antibody into the specimen introduction portion
· A step of applying a centrifugal force to the analysis chip and destroying the blocking portion using light or heat to: (1) first cause the specimen to reach the reaction portion; after the specimen reaches the reaction portion, (2) cause a washing solution introduced into the washing solution storage portion to reach the reaction portion and discharge the washing solution from the reaction portion; and then (3) cause a solution, in which the dry reagent has been dissolved in a dissolving solution introduced into the dissolving solution storage portion, to reach the reaction portion

### · A step of measuring an absorbance of the liquid stored in the reaction portion

More specifically, in the analysis chip including the blocking portion only in the sub flow path b3 of the third flow path 53 as shown in FIG. 6, it is preferable that the first flow path and the second flow path are designed to be different from each other in at least one of the cross-sectional area, the length, and the hydrophilicity of the flow path inner wall, as described in the first embodiment and the second embodiment. The washing of the reaction portion 16 after the specimen has been introduced into the reaction portion 16 or the dissolution of the dry reagent (up to before the introduction into the reaction portion 16) need only be performed by appropriately applying a centrifugal force as described in the first embodiment and the second embodiment. That is, (3) of FIG. 3 may be performed.

After that, the sub flow path b3 is opened by applying light or heat to at least the blocking portion, and a centrifugal force of an appropriate magnitude is applied to the analysis chip. Consequently, the solution obtained by dissolving the dry reagent can be introduced into the reaction portion 16.

In the analysis chip shown in FIG. 6, the blocking portion is provided only in the sub flow path b3 of the third flow path 53, but the blocking portion may be provided in other flow paths.

For example, in FIG. 6, the blocking portion may be provided not only in the sub flow path b3 of the third flow path 53, but also in three sub flow paths a of the second flow path 52 on the downstream side of three measurement portions aa. In this case, the analysis can be performed in the following steps (order) .
- One of the three blocking portions present in the second flow path 52 is destroyed. Then, when a centrifugal force is applied to the analysis chip, the washing solution flows from one measurement portion aa to the reaction portion 16.
- The second blocking portion of the three blocking portions present in the second flow path 52 is destroyed. Then, when a centrifugal force is applied to the analysis chip, the washing solution flows from the second measurement portion aa to the reaction portion 16.
- The third blocking portion of the three blocking portions present in the second flow path 52 is destroyed. Then, when a centrifugal force is applied to the analysis chip, the washing solution flows from the third measurement portion aa to the reaction portion 16.
- The blocking portion present in the sub flow path b3 in the third flow path 53 is destroyed. Then, when a centrifugal force is applied to the analysis chip, the solution obtained by dissolving the dry reagent can be introduced into the reaction portion 16.

The blocking portion that can be destroyed by light or heat can be provided, for example, by forming a recessed portion corresponding to the flow path using an injection molding method and then coating a part of the recessed portion with an appropriate resin material. The blocking portion that can be destroyed by light or heat can be formed by appropriately using a known technology. For example, in a WEB column of the Japan Society of Applied Physics (https://www.jsap.or.jp/columns-covid19/covid19_4-2-2), there is a description such as "the resin valve that encloses the reagent storage chamber is opened ...". The technology of this resin valve can be applied to the third embodiment. In addition, the blocking portion can also be made of gelatin, hydroxypropyl cellulose, or the like. This can also be referred to in the description of Japanese Unexamined Patent Publication No. 2008-151773.

Of course, the method of forming the blocking portion and the material constituting the blocking portion are not particularly limited. For example, a blocking portion can be formed by using a resin material having a melting point lower than that of the plate-like member 1 and the lid member 3, and when the analysis chip is used, the blocking portion can be destroyed by heating at least the blocking portion to a temperature equal to or higher than the melting point of the blocking portion and lower than the melting points of the plate-like member 1 and the lid member 3. Alternatively, instead of heating, destruction by irradiation with laser light is also conceivable.

### <Analysis Protocol (Reference)>

The first to third embodiments mentioned above implement an analysis protocol using a microplate provided with wells, as exemplified below, within the chip. Specifically, the following sections 1 and 2 can be performed by processing the reaction portion 16 in advance in the analysis chip (before use). Then, as described in the first to third embodiments, the following sections 3 and 4 can be performed by using a centrifugal force or the like.

### 1. Immobilization of Primary Antibody

(1) A primary antibody solution is prepared by diluting a primary antibody with a carbonate buffer to 10 µg/mL.
(2) 100 µL of the primary antibody solution is added to each well of the microplate and is shaken at a room temperature for 15 minutes under the condition of 400 rpm. In this manner, the primary antibody is immobilized in the microplate well.
(3) The inside of the well is washed three times with a solution containing 0.05% by mass of polyethylene glycol sorbitan monolaurate (commercial name: Tween 20), which is a nonionic surfactant, in Tris-buffered saline (TBS).

### 2. Blocking

(1) 300 µL of a TBS solution containing 1 % by mass of bovine serum albumin (BSA) is added to each well and allowed to stand at a room temperature for 30 minutes.
(2) After that, the inside of each well is washed three times by using 300 µL per well of a solution containing 0.05% by mass of Tween 20 in TBS.

### 3. Antigen

(1) The antigen is diluted to a predetermined concentration (as appropriate). Specifically, an alkaline phosphatase (ALP)-labeled secondary antibody is prepared by using a diluent containing 0.1% by mass of BSA in TBS such that the concentration of the antigen is about 100 ng/mL. The same solution as the solution used for diluting the antigen is used for the blank.
(2) 100 µL of the diluted antigen is added to each well. Then, the mixture is shaken at a room temperature for 45 minutes under the condition of 400 rpm.
(3) After that, the inside of each well is washed three times by using 300 µL per well of a liquid containing 0.05% by mass of Tween 20 in TBS.

### 4. Measurement

The [R-1 freeze-dried product] and [R-2 freeze-dried product] shown below are mixed in equal amounts to form a mixture in the manner described in [R-1 and R-2 dissolving solutions] below. 100 µL of this mixture is added to each well, and the color development reaction is allowed to proceed. The color development reaction is performed at 37°C. Then, the absorbance measurement is started. Specifically, the absorbance at a wavelength of 405 nm is measured. The absorbance measurement is performed once every 5 minutes, for a total of 12 measurements.

### [R-1 Freeze-Dried Product]

A product obtained by freeze-drying a composition having the following composition
- 10 mM NADH
- 4 mM substrate (A3P: Androsterone 3-phosphate)
- 20 mM Tris-HCl Buffer (containing a stabilizer, pH 9.25)

### [R-2 Freeze-Dried Product]

A product obtained by freeze-drying a composition having the following composition
· 20 mM thio-NAD
· 100U/mL 3α-HSD
· 20 mM Tris-HCl Buffer (containing a stabilizer, pH 7.5)

### [R-1 and R-2 Dissolving Solutions]

Each of the above-described [R-1 freeze-dried product] and [R-2 freeze-dried product] is dissolved in 3.75 mL of 100 mM Tris-HCl buffer (pH 9.5).

The concentrations of the reagents in the mixture are as follows.
1 mM NADH
0.4 mM substrate (A3P)
2 mM thio-NAD
10U/mL 3α-HSD

For reference, the present inventor has confirmed that influenza virus A can be detected with an ultra-high sensitivity of 3.125 pfu/mL by the above-described protocol.

The analysis chips of the first to third embodiments can easily perform such ultra-high-sensitivity measurement.

Hereinabove, the embodiments of the present invention have been described, and these are examples of the present invention. Various configurations other than those described above can be employed. Additionally, the present invention is not limited to the embodiments mentioned above, and modifications, improvements, and the like within the scope that can achieve the object of the present invention are included in the present invention.

This application claims priority based on Japanese Patent Application No. 2023-036622 filed on March 9, 2023, the entire disclosure of which is incorporated herein by reference.

### REFERENCE SIGNS LIST

- 1: plate-like member
- 3: lid member
- 5A: washing solution package
- 5B: dissolving solution package
- 10: specimen introduction portion
- 12: washing solution storage portion
- 14: dissolving solution storage portion
- 16: reaction portion
- 18: dry reagent enclosing portion
- 20: waste liquid storage portion
- 22A: mirror
- 22B: mirror
- 22C: mirror
- 51: first flow path
- 52: second flow path
- 53: third flow path
- a: sub flow path
- aa: measurement portion
- b1: sub flow path
- b2: sub flow path
- b3: sub flow path
- c1: first dry reagent enclosing portion
- c2: second dry reagent enclosing portion
- m: mixing portion

## Claims

1. An analysis chip comprising:
a specimen introduction portion into which a liquid specimen containing a protein or nucleic acid labeled with an enzyme-labeled antibody, or a liquid specimen containing an enzyme-labeled antibody and a protein or nucleic acid is introduced;
a washing solution storage portion;
a dissolving solution storage portion;
a reaction portion provided with an immobilized antibody or nucleic acid;
a first flow path that connects the specimen introduction portion and the reaction portion;
a second flow path that connects the washing solution storage portion and the reaction portion; and
a third flow path that connects the dissolving solution storage portion and the reaction portion and that includes a dry reagent enclosing portion provided midway in the flow path, the dry reagent enclosing portion enclosing a dry reagent containing NADH, a substrate for an enzyme in the enzyme-labeled antibody, thio-NAD, and dehydrogenase,
wherein, by making the first flow path, the second flow path, and the third flow path different from each other in at least one of a cross-sectional area, a length, and hydrophilicity of a flow path inner wall,
when a predetermined centrifugal force is applied to the analysis chip: (1) the liquid specimen introduced into the specimen introduction portion is first caused to reach the reaction portion; after the specimen reaches the reaction portion, (2) a washing solution introduced into the washing solution storage portion is caused to reach the reaction portion; and after the washing solution reaches the reaction portion, (3) a solution, in which the dry reagent has been dissolved in a dissolving solution introduced into the dissolving solution storage portion, is caused to reach the reaction portion.

2. The analysis chip according to Claim 1,
wherein the first flow path, the second flow path, and the third flow path are different from each other in at least one of the cross-sectional area and the hydrophilicity of the flow path inner wall, and
a magnitude of the predetermined centrifugal force increases at least once as time elapses.

3. The analysis chip according to Claim 1,
wherein the first flow path, the second flow path, and the third flow path are different from each other in at least the length, and
a magnitude of the predetermined centrifugal force is substantially constant over time.

4. An analysis chip comprising:
a specimen introduction portion into which a liquid specimen containing a protein or nucleic acid labeled with an enzyme-labeled antibody, or a liquid specimen containing an enzyme-labeled antibody and a protein or nucleic acid is introduced;
a washing solution storage portion;
a dissolving solution storage portion;
a reaction portion provided with an immobilized antibody or nucleic acid;
a first flow path that connects the specimen introduction portion and the reaction portion;
a second flow path that connects the washing solution storage portion and the reaction portion; and
a third flow path that connects the dissolving solution storage portion and the reaction portion and that includes a dry reagent enclosing portion provided midway in the flow path, the dry reagent enclosing portion enclosing a dry reagent containing NADH, a substrate for an enzyme in the enzyme-labeled antibody, thio-NAD, and dehydrogenase,
wherein at least one of the second flow path and the third flow path includes a blocking portion provided midway, and the blocking portion is configured to prevent a liquid from passing through, and
the blocking portion is destroyable by light or heat, and the liquid is allowed to pass through the flow path when the blocking portion is destroyed.

5. The analysis chip according to any one of Claims 1, 2, and 4,
wherein the second flow path includes three to five sub flow paths a that connect the washing solution storage portion and the reaction portion, and
the sub flow paths are different from each other in at least one of a cross-sectional area, a length, and hydrophilicity of a flow path inner wall.

6. The analysis chip according to any one of Claims 1, 2, and 4,
wherein the third flow path includes two sub flow paths, a sub flow path b1 and a sub flow path b2, extending from the dissolving solution storage portion, a mixing portion to which the sub flow path b1 and the sub flow path b2 are connected, a sub flow path b3 that connects the mixing portion to the reaction portion, a first dry reagent enclosing portion c1 provided midway in the sub flow path b1, and a second dry reagent enclosing portion c2 provided midway in the sub flow path b2, and
one of the dry reagent enclosing portion c1 and the dry reagent enclosing portion c2 encloses NADH and a substrate for the enzyme in the enzyme-labeled antibody, and the other of the dry reagent enclosing portion c1 and the dry reagent enclosing portion c2 encloses thio-NAD and dehydrogenase.

7. The analysis chip according to any one of Claims 1, 2, and 4, further comprising:
a waste liquid storage portion; and
a fourth flow path that connects the reaction portion and the waste liquid storage portion.

8. The analysis chip according to any one of Claims 1, 2, and 4, further comprising:
a washing solution package in which the washing solution is enclosed with a first packaging material,
wherein the washing solution package is configured such that the washing solution is supplied to the washing solution storage portion when the first packaging material is destroyed.

9. The analysis chip according to Claim 8,
wherein the washing solution is a buffer solution containing a surfactant.

10. The analysis chip according to any one of Claims 1, 2, and 4, further comprising:
a dissolving solution package in which the dissolving solution is enclosed with a second packaging material,
wherein the dissolving solution package is configured such that the dissolving solution is supplied to the dissolving solution storage portion when the second packaging material is destroyed.

11. The analysis chip according to Claim 10,
wherein the dissolving solution is a buffer solution.

12. An analysis method for a specimen, comprising:
a step of introducing a liquid specimen containing a protein or nucleic acid labeled with an enzyme-labeled antibody into the specimen introduction portion in the analysis chip according to Claim 1 or 2;
a step of applying a centrifugal force to the analysis chip to: (1) first cause the specimen to reach the reaction portion; after the specimen reaches the reaction portion, (2) cause a washing solution introduced into the washing solution storage portion to reach the reaction portion and discharge the washing solution from the reaction portion; and then (3) cause a solution, in which the dry reagent has been dissolved in a dissolving solution introduced into the dissolving solution storage portion, to reach the reaction portion; and
a step of measuring an absorbance of a liquid stored in the reaction portion.

13. An analysis method for a specimen, comprising:
a step of introducing a liquid specimen containing a protein or nucleic acid labeled with an enzyme-labeled antibody into the specimen introduction portion in the analysis chip according to Claim 4;
a step of applying a centrifugal force to the analysis chip and destroying the blocking portion using light or heat to: (1) first cause the specimen to reach the reaction portion; after the specimen reaches the reaction portion, (2) cause a washing solution introduced into the washing solution storage portion to reach the reaction portion and discharge the washing solution from the reaction portion; and then (3) cause a solution, in which the dry reagent has been dissolved in a dissolving solution introduced into the dissolving solution storage portion, to reach the reaction portion; and
a step of measuring an absorbance of a liquid stored in the reaction portion.

14. An analysis system for a specimen, comprising:
the analysis chip according to Claim 1 or 2;
a centrifugal force applying unit configured to apply a centrifugal force to the analysis chip; and
an absorbance measuring unit configured to measure an absorbance of a liquid stored in the reaction portion in the analysis chip.

15. An analysis system for a specimen, comprising:
the analysis chip according to Claim 4;
a centrifugal force applying unit configured to apply a centrifugal force to the analysis chip;
a light source or a heat source configured to destroy the blocking portion in the analysis chip; and
an absorbance measuring unit configured to measure an absorbance of a liquid stored in the reaction portion in the analysis chip.
